# EUROPEAN PATENT APPLICATION

(11) **EP 3 320 841 A1**
(43) Date of publication of application: **16.05.2018**
(21) Application number: 15882383.1
(22) Date of filing: 08.09.2015
(51) Int. Cl.: A61B 5/107, A61B 5/16, A61B 3/11

(54) **USER HEALTH MONITORING METHOD, MONITORING DEVICE, AND MONITORING TERMINAL**

(30) Priority: 10.07.2015 CN 201510404221
(71) Applicant: ZTE Corporation, Shenzhen, Guangdong 518057 (CN)
(72) Inventor: LIU, Fengpeng, Shenzhen Guangdong 518057 (CN); LIU, Dongmei, Shenzhen Guangdong 518057 (CN)
(74) Representative: HGF Limited
(86) International application number: PCT/CN2015/089188
(87) International publication number: WO 2016/131244

(57) **Abstract**

A method for monitoring user health, a device for monitoring user health, and a terminal for monitoring user health are provided. A method for monitoring user health includes: acquiring an illuminance of an environment, where the environment is an environment in which a user is currently located; acquiring a first pupil size when the user is located in the environment; correcting the first pupil size on the basis of the illuminance to acquire a second pupil size; comparing the second pupil size with a reference pupil size to determine a change rate of a pupil size of the user; and determining a health state of the user on the basis of the change rate of the pupil size. The problem that the terminal device may not monitor user's health comprehensively can be solved and the effect of monitoring user's health comprehensively can be achieved.

## Description

### Technical Field

The present disclosure relates to the field of health monitoring, and in particular relates to a method for monitoring user health, a device for monitoring user health and a terminal for monitoring user health.

### Background

In a terminal device, piggybacking with medical health monitoring function is a subject in recent years, but feasible solutions are few. In the existing terminal medical health solutions, user health can only be determined by detecting blood oxygen and heart rate, deeper and more professional solutions are not yet realized. Further the solutions by detecting blood oxygen and heart rate are complex and high cost needs to be increased for the terminal device.

Aiming at the problem of incomplete health monitoring of users in related art, no effective solution has been put forward currently.

### Summary

The present disclosure provides a method for monitoring user health, a device for monitoring user health and a terminal for monitoring user health, at least solves the problem of incomplete health monitoring of the user in the related art.

According to an embodiment of the present disclosure, the present disclosure provides a method for monitoring user health, including: acquiring an illuminance of an environment, herein the environment is an environment in which a user is currently located; acquiring a first pupil size when the user is located in the environment; correcting the first pupil size on the basis of the illuminance to acquire a second pupil size; comparing the second pupil size with a reference pupil size to determine a change rate of a pupil size of the user; and determining a health state of the user on the basis of the change rate of the pupil size.

According to an embodiment of the present disclosure, correcting the first pupil size on the basis of the illuminance to acquire a second pupil size includes: calculating a change size of pupil on the basis of effects on the first pupil size under the illuminance; and calculating the second pupil size on the basis of the change size of pupil and the first pupil size.

According to an embodiment of the present disclosure, calculating a change size of pupil on the basis of effects on the first pupil size under the illuminance includes: calculating the change size of pupil according to a formula *Y*=(*L*₁*-L*₂)**Q,* herein *Y* is the change size of pupil, *L*₁ is the illuminance, *L*₂ is a reference illuminance, *Q* is a change coefficient of pupil size; calculating the second pupil size on the basis of the change size of pupil and the first pupil size includes: calculating the second pupil size according to a formula *A*=*X*+*Y,* herein *A* is the second pupil size, *X* is the first pupil size.

According to an embodiment of the present disclosure, comparing the second pupil size with a reference pupil size to determine a change rate of a pupil size of the user includes: calculating the change rate of the pupil size according to a formula *W=*(*A-B)*/*B,* herein *W* is the change rate of the pupil size, *B* is the reference pupil size, *A* is the second pupil size.

According to an embodiment of the present disclosure, determining a health state of the user on the basis of the change rate of the pupil size includes: determining the health state of the user to be a first state in the case that the change rate of the pupil size is greater than a preset threshold of change rate; determining the health state of the user to be a second state in the case that the change rate of the pupil size is less than the preset threshold of change rate.

According to an embodiment of the present disclosure, determining a health state of the user on the basis of the change rate of the pupil size includes: determining the health state of the user to be a first state in the case that the change rate of the pupil size is within a range of a first preset change rate; determining the health state of the user to be a second state in the case that the change rate of the pupil size is within a range of a second preset change rate; determining the health state of the user to be a three state in the case that the change rate of the pupil size is within a range of a three preset change rate.

According to an embodiment of the present disclosure, after determining a health state of the user on the basis of the change rate of the pupil size, the method further includes: recording the health state of the user; sending an alert message to a terminal held by the user, herein the alert message includes the health state of the user.

According to another embodiment of the present disclosure, the present disclosure provides a device for monitoring user health, including: a first acquisition module, configured to acquire an illuminance of an environment, herein the environment is an environment in which a user is currently located; a second acquisition module, configured to acquire a first pupil size when the user is located in the environment; a correction module, configured to correct the first pupil size on the basis of the illuminance to acquire a second pupil size; a comparison module, configured to compare the second pupil size with a reference pupil size to determine a change rate of a pupil size of the user; and a determination module, configured to determine a health state of the user on the basis of the change rate of the pupil size.

According to an embodiment of the present disclosure, the correction module includes: a first calculation unit, configured to calculate a change size of pupil on the basis of effects on the first pupil size under the illuminance; a second calculation unit, configured to calculate the second pupil size on the basis of the change size of pupil and the first pupil size.

According to an embodiment of the present disclosure, the first calculation unit includes: a first calculation subunit, configured to calculate the change size of pupil according to a formula *Y*=(*L*₁*-L*₂)**Q*, where *Y* is the change size of pupil, *L*₁ is the illuminance, *L*₂ is a reference illuminance, *Q* is a change coefficient of pupil size; the second calculation unit includes: a second calculation subunit, configured to calculate the second pupil size according to a formula *A*=*X*+*Y,* where *A* is the second pupil size, *X* is the first pupil size.

According to an embodiment of the present disclosure, the comparison module includes: a third calculation unit, configured to calculate the change rate of the pupil size according to a formula *W*=(*A-B*)/*B,* where *W* is the change rate of the pupil size, *B* is the reference pupil size, *A* is the second pupil size.

According to an embodiment of the present disclosure, the determination module includes: a first determination unit, configured to determine the health state of the user to be a first state in the case that the change rate of the pupil size is greater than a preset threshold of change rate; a second determination unit, configured to determine the health state of the user to be a second state in the case that the change rate of the pupil size is less than the preset threshold of change rate.

According to an embodiment of the present disclosure, the determination module further includes: a third determination unit, configured to determine the health state of the user to be a first state in the case that the change rate of the pupil size is within a range of a first preset change rate; a fourth determination unit, configured to determine the health state of the user to be a second state in the case that the change rate of the pupil size is within a range of a second preset change rate; a fifth determination unit, configured to determine the health state of the user to be a three state in the case that the change rate of the pupil size is within a range of a three preset change rate.

According to an embodiment of the present disclosure, the device further includes: a recording unit, configured to record the health state of the user; a sending unit, configured to send an alert message to a terminal held by the user, herein the alert message includes the health state of the user.

According to another embodiment of the present disclosure, the present disclosure provide a terminal for monitoring user health, including: a first sensor, configured to acquire an illuminance of an environment, herein the environment is an environment in which a user is currently located; a second sensor, configured to acquire a first pupil size when the user is located in the environment; a processor, connected with the first sensor and the second sensor, configured to correct the first pupil size on the basis of the illuminance to acquire a second pupil size, compare the second pupil size with a reference pupil size to determine a change rate of a pupil size of the user and determine a health state of the user on the basis of the change rate of the pupil size.

By the present disclosure, the illuminance of the environment is acquired, where the environment is an environment in which a user is currently located, the first pupil size is acquired when the user is located in the environment, the first pupil size is corrected on the basis of the illuminance to acquire a second pupil size; the second pupil size is compared with a reference pupil size to determine a change rate of a pupil size of the user; and the health state of the user is determined on the basis of the change rate of the pupil size. Thereby the problem that the terminal device may not monitor user health comprehensively can be solved and the effect of monitoring user health comprehensively can be achieved.

### Brief Description of Drawings

The accompanying drawings are included to provide a further understanding of the disclosure, and constitute as a part of this application, the illustrative embodiments and the description thereof in the disclosure are given solely for the purpose of illustration and are not to be construed as improper limitations of the present disclosure. In the drawings:
Fig.1 is a flow chart illustrating a method for monitoring user health according to an embodiment of the present disclosure.
Fig.2 is a block diagram illustrating a device for monitoring user health according to an embodiment of the present disclosure.
Fig.3 is a schematic diagram illustrating a terminal for monitoring user health according to an embodiment of the present disclosure.
Fig.4 is a flow chart illustrating a terminal monitoring user health according to a preferred embodiment of the present disclosure.
Fig.5 is a flow chart illustrating a method for monitoring user health according to a preferred embodiment of the present disclosure.

### Detailed Description

The disclosure will be explained below with reference to the accompanying drawings and in combination with embodiments, it is noted that in case of no conflict, the embodiments of the present application and the features in the embodiments may be mutually combined.

It is noted that the terms "first", "second" and the like in the description and claims of the present disclosure, are used for distinguishing between similar objects and not necessarily for describing a sequential or chronological order.

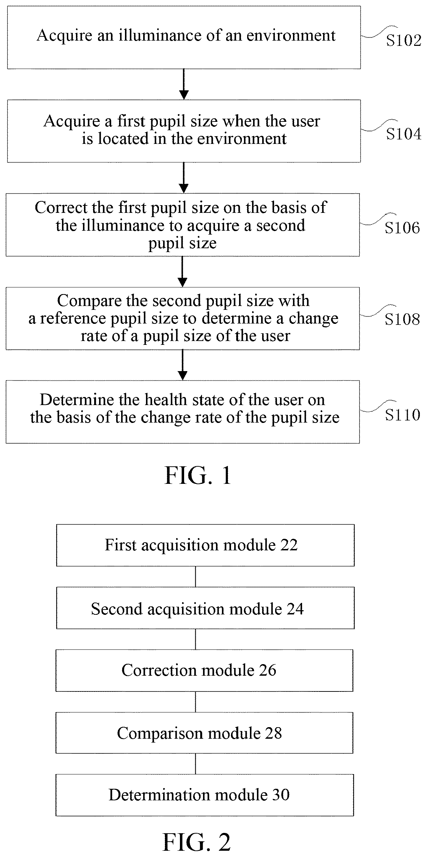

According to an embodiment of the present disclosure, the present disclosure provides a method for monitoring user health. Fig.1 is a flow chart illustrating the method for monitoring user health according to an embodiment of the present disclosure. As shown in Fig. 1, the flow includes the following steps.

In step S102, the illuminance of the environment is acquired, where the environment is an environment in which the user is currently located.

In step S104, a first pupil size is acquired when the user is located in the environment.

In step S106, the first pupil size is corrected on the basis of the illuminance to acquire a second pupil size.

In step S108, the second pupil size is compared with a reference pupil size to determine a change rate of a pupil size of the user.

In step S110, the health state of the user is determined on the basis of the change rate of the pupil size.

By the steps described above, the illuminance of the environment is acquired, where the user is currently located in the environment, the first pupil size is acquired when the user is located in the environment, the first pupil size is corrected on the basis of the illuminance to acquire a second pupil size; the second pupil size is compared with a reference pupil size to determine a change rate of a pupil size of the user; and the health state of the user is determined on the basis of the change rate of the pupil size. The illuminance of the environment where the user is currently located and the user's pupil size when the user is located in the environment are acquired. Then, the pupil size is corrected to obtain a more accurate pupil size, so as to reduce the effect of the illuminance on the pupil size of the user. Finally, the change rate of the user's pupil size is determined according to the more accurate pupil size and the reference pupil size under the reference illuminance, so that the user's health state is determined. Thereby the problem that the terminal device may not monitor user's health comprehensively can be solved, and the effect of monitoring user's health comprehensively can be achieved.

In the present embodiment, correcting the first pupil size on the basis of the illuminance to acquire the second pupil size may be achieved by the step S1061 and step S1063, where the step S1061 and step S1063 are as follows.

In step S1061, a change size of pupil is calculated on the basis of effects on the first pupil size under the illuminance.

Optionally, the change size of pupil may be calculated according to the formula *Y*=(*L*₁*-L*₂)**Q*, where *Y* is the change size of pupil, *L*₁ is the illuminance, *L*₂ is a reference illuminance, *Q* is a change coefficient of pupil size.

The change coefficient of pupil size *Q* is related to the reference illuminance *L*₂ and is derived from big data statistics, and the specific value of reference illuminance *L*₂ may be determined by user demands. For example, the change coefficient of pupil size is 0.002mm/lux if the reference illuminance is 500 lux.

In step S1063, a second pupil size is calculated on the basis of the change size of pupil and the first pupil size.

Optionally, the second pupil size may be calculated according to the formula *A*=*X*+*Y,* where *A* is the second pupil size, *X* is the first pupil size.

In this embodiment, the change rate of the pupil size may be calculated according to the formula *W*=(*A-B*)/*B,* where *W* is the change rate of the pupil size, *B* is the reference pupil size, *A* is the second pupil size.

According to embodiments of the present disclosure, there are two ways of determining the health state of the user on the basis of the change rate of the pupil size, detailed descriptions are as follows.

Mode 1: in the case that the change rate of the pupil size is greater than a preset threshold of change rate, it is determined that the health state of the user is a first state; in the case that the change rate of the pupil size is less than the preset threshold of change rate, it is determined that the health state of the user is a second state. In the mode 1, the preset threshold of change rate can be 0, the first state is healthy, the second state is unhealthy.

Mode 2: in the case that the change rate of the pupil size is in a range of the first preset change rate, it is determined that the health state of the user is a first state; in the case that the change rate of the pupil size is in a range of the second preset change rate, it is determined that the health state of the user is a second state; in the case that the change rate of the pupil size is in a range of the third preset change rate, it is determined that the health state of the user is a third state. In the mode 2, the first state is healthy, the second state is sub-health, the third state is unhealthy. The first preset threshold of change rate is greater than the second preset threshold of change rate, which is greater than the third preset threshold of change rate, and the first preset threshold of change rate, second preset threshold of change rate and third preset threshold of change rate can be configured according to the user demands For example, the range of the first preset change rate is greater than 0; the range of the second preset change rate is (0,-10%], the range of the third preset change rate is less than -10%.

According to an embodiment of the present disclosure, in order to make the user know the health condition timely, after the health state of the user is determined on the basis of the change rate of the pupil size, the method further includes the following steps.

In step S112, the health state of the user is recorded. The health states of the user at different times are known by recording the health states of the user, so as to form a health state database of the user which may provide data for further statistical analysis of the health state of the user.

In step S 114, an alert message is sent to the terminal held by the user, herein the alert message includes the health state of the user. Specifically, the terminal can be any terminal including a laptop computer, a cellular phone, a tablet computer and so on, which may receive the alert message.

According to an embodiment of the present disclosure, the terminal may send the alert message, including the health state of the user, to the terminal held by the user, so that the user can know their health state timely.

Optionally, the alert message can be sent fixedly at a time interval (for example, once a day) preset by the user, the alert message may always be sent fixedly at a preset time interval regardless of the health state of the user. The alert message also can be sent according to the health state of the user. For example, the alert message may not be sent when the health state of the user is healthy, and the alert message may be sent every 4 hours when the health state of the user is unhealthy.

The present embodiment also provides a device for monitoring user health, the device is used for achieving the above embodiments and preferred embodiment, the content described above will not be commented any more here. The term "module" may be a combination of hardware and/or software which can implement a preset function. Although the device described in below embodiments may be preferably implemented by software, it can be conceived that the device may be implemented by hardware or a combination of hardware and software.

Fig.2 is a block diagram illustrating a device for monitoring user health according to an embodiment of the present disclosure. As shown in Fig. 2, the device includes the following modules a first acquisition module 22, a second acquisition module 24, a correction module 26, a comparison module 28 and a determination module 30.

The first acquisition module 22 is configured to acquire an illuminance of an environment, where the environment is an environment in which a user is currently located.

The second acquisition module 24 is configured to acquire a first pupil size when the user is located in the environment.

The correction module 26 is configured to correct the first pupil size on the basis of the illuminance to acquire a second pupil size.

The comparison module 28 is configured to compare the second pupil size with a reference pupil size to determine a change rate of a pupil size of the user.

The determination module 30 is configured to determine a health state of the user on the basis of the change rate of the pupil size.

By the device described above, the illuminance of the environment is acquired, where the user is currently located in the environment, the first pupil size is acquired when the user is located in the environment, the first pupil size is corrected on the basis of the illuminance to acquire a second pupil size; the second pupil size is compared with a reference pupil size to determine a change rate of a pupil size of the user; and the health state of the user is determined on the basis of the change rate of the pupil size. The illuminance of the environment where the user is currently located and the user's pupil size when the user is located in the environment are acquired. Then, the pupil size is corrected to obtain a more accurate pupil size, so as to reduce the effect of the illuminance on the pupil size of the user. Finally, the change rate of the user's pupil size is determined according to the more accurate pupil size and the reference pupil size under the reference illuminance, so that the user's health state is determined, thereby the problem that the terminal device may not monitor user's health comprehensively can be solved, and the effect of monitoring user's health comprehensively can be achieved.

In this embodiment, the correction module 26 includes a first calculation unit and a second calculation unit.

The first calculation unit is configured to calculate a change size of pupil on the basis of effects on the first pupil size under the illuminance.

The second calculation unit is configured to calculate the second pupil size on the basis of the change size of pupil and the first pupil size.

In this embodiment, the first calculation unit includes a first calculation subunit, the second calculation unit includes a second calculation subunit. The first calculation subunit is configured to calculate the change size of pupil according to the formula *Y*=(*L*₁*-L*₂)**Q*, where *Y* is the change size of pupil, *L*₁ is the illuminance, *L*₂ is a reference illuminance, *Q* is a change coefficient of pupil size. The second calculation subunit is configured to calculate the second pupil size on the basis of the change size of pupil and the first pupil size, including: calculating the second pupil size according to the formula *A*=*X*+*Y,* where *A* is the second pupil size, *X* is the first pupil size.

In this embodiment, the comparison module 28 includes a third calculation unit, the third calculation unit is configured to calculate the change rate of the pupil size according to the formula *W*=(*A-B*)/*B,* where *W* is the change rate of the pupil size, *B* is the reference pupil size, *A* is the second pupil size.

In this embodiment, the determination module 30 includes a first determination unit and a second determination unit, where the first determination unit is configured to determine that the health state of the user is a first state in the case that the change rate of the pupil size is greater than a preset threshold of change rate. The second determination unit is configured to determine that the health state of the user is a second state in the case that the change rate of the pupil size is less than the preset threshold of change rate. Specifically, the preset threshold of change rate can be configured according to the demands. For example, the preset threshold of change rate can be 0, the first state represents healthy, the second state represents unhealthy.

In this embodiment, the determination module 30 further includes a third determination unit, a fourth determination unit and a fifth determination unit, herein, the third determination unit is configured to determine that the health state of the user is a first state in the case that the change rate of the pupil size is in a range of the first preset change rate. The fourth determination unit is configured to determine that the health state of the user is a second state in the case that the change rate of the pupil size is in a range of the second preset change rate. The fifth determination unit is configured to determine that the health state of the user is a three state in the case that the change rate of the pupil size is in a range of the third preset change rate. Specifically, the first state represents healthy, the second state represents sub-health, the third state represents unhealthy. The first preset threshold of change rate is greater than the second preset threshold of change rate, which is greater than the third preset threshold of change rate, and the first preset threshold of change rate, second preset threshold of change rate and third preset threshold of change rate can be configured according to the user demands. For example, the range of the first preset change rate is greater than 0; the range of the second preset change rate is (0,-10%], the range of the third preset change rate is less than -10%.

In this embodiment, in order to keep users be informed of their health state timely, the device further includes a recording unit and a sending unit.

The recording unit is configured to record the health state of the user after the health state of the user is determined on the basis of the change rate of the pupil size. The health states of the user at different times are known by recording the health states of the user, so as to form a health state database of the user which may provide data for further statistical analysis of the health state of the user.

The sending unit is configured to send an alert message to the terminal held by the user, where the alert message includes the health state of the user. Specifically, the terminal can be any terminal including a laptop computer, a cellular phone, a tablet computer and so on, which may receive the alert message.

According to an embodiment of the present disclosure, the terminal may send the alert message, including the health state of the user, to the terminal held by the user, so that the users can know their health state timely.

Optionally, the alert message can be sent fixedly at a time interval (for example, once a day) preset by the user, the alert message may always be sent fixedly at a preset time interval regardless of the health state of the user. The alert message also can be sent according to the health state of the user. For example, the alert message may not be sent when the health state of the user is healthy, and the alert message may be sent every 4 hours when the health state of the user is unhealthy.

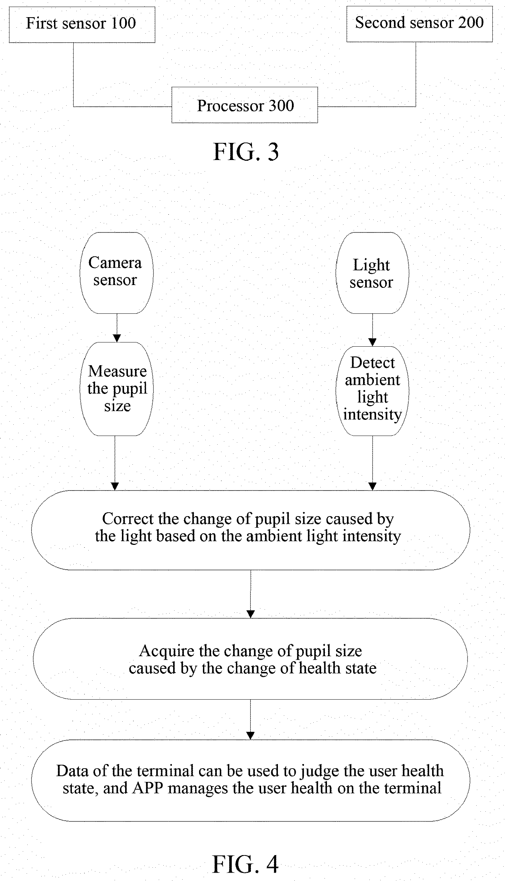

According to an embodiment of the present disclosure, the present disclosure provides a terminal for monitoring user health. Fig.3 is a schematic diagram illustrating a terminal for monitoring user health according to an embodiment of the present disclosure. As shown in Fig.3, the terminal includes a first sensor 100, a second sensor 200 and a processor 300.

The first sensor 100 is configured to acquire an illuminance of an environment, where the environment is an environment in which a user is currently located. Specifically, the first sensor can also be called a light sensor.

The second sensor 200 is configured to acquire a first pupil size when the user is located in the environment. Specifically, the second sensor can also be called a camera sensor.

The processor 300 is connected with both the first sensor 100 and the second sensor 200, which is configured to correct the first pupil size on the basis of the illuminance to acquire a second pupil size, compare the second pupil size with a reference pupil size to determine a change rate of a pupil size of the user, and determine a health state of the user on the basis of the change rate of the pupil size.

By the terminal described above, the illuminance of the environment is acquired, where the user is currently located in the environment, the first pupil size is acquired when the user is located in the environment, the first pupil size is corrected on the basis of the illuminance to acquire a second pupil size; the second pupil size is compared with a reference pupil size to determine a change rate of a pupil size of the user; and the health state of the user is determined on the basis of the change rate of the pupil size. The illuminance of the environment where the user is currently located and the user's pupil size when the user is located in the environment are acquired. Then, the pupil size is corrected to obtain a more accurate pupil size, so as to reduce the effect of the illuminance on the pupil size of the user. Finally, the change rate of the user's pupil size is determined according to the more accurate pupil size and the reference pupil size under the reference illuminance, so that the user's health state is determined, thereby the problem that the terminal device may not monitor user's health comprehensively can be solved and the effect of monitoring user's health comprehensively can be achieved. In addition, because two sensors are used in this embodiment to collect the required data, the way for monitoring user health provided in this application significantly reduces the additional costs of the terminal compared to the relevant technology.

Fig.4 is a flow chart illustrating a terminal monitoring user health according to a preferred embodiment of the present disclosure. As shown in Fig.4, in this monitoring terminal, the "camera sensor" and the "light sensor" are taken as examples. Data related to user health is collected and then analyzed and compared by terminal to determine user's health; and then user's health management would be performed in an APP. The camera sensor is used as a sensor for collecting graphic images, in this embodiment, the graphic image is collected to collect the size of the pupil, and the size of the pupil represents user health state. For instance, compared with the pupil size in a general case, the pupil size becomes smaller in panic and anxiety; while pupil becomes bigger in pleasure. The light sensor can capture ambient light (illuminance) in that pupil size would change due to light changes. Specifically, if human eyes experience a greater illuminance, the pupil will have to shrink; while human eyes experience a less illuminance, the pupil will have to enlarge. Therefore, a camera sensor is mainly used in the monitoring terminal for collecting the pupil size as the emotion acquisition scale, with the cooperation of a light sensor to exclude pupil changes caused by light changes, so as to determine the user health state accurately.

Specifically, the process of determining a user health is as follows: supposing that, the pupil diameter measured by a camera sensor (the first pupil size in the above embodiment) is defined as X in millimeters; pupil changes (change of pupil size in the above embodiment) caused by the illuminance is defined as Y in millimeters, and the corrected pupil size is defined as A in millimeters. Then, A = X + Y. A represents the second pupil size in the above embodiment. The illuminance that can be felt by the human eyes is defined as L in lux, the reference illuminance is defined as 500 lux and the change coefficient of illuminance and pupil size is defined as Q in millimeters/lux. The coefficient Q comes from big data statistic, and represents by how many millimeters the pupil size may change when the illuminance changes by one 1 lux. Then, Y = (L-500)*Q. A = X + (L-500)*Q. A is a real pupil size reflecting a user health state. Supposing, the standard pupil size under the reference illuminance and in calmness is defined as B in millimeters, and the change rate of pupil size is defined as W, then W = (A-B) / B. According to the corrected change rate of pupil size and big data statistics, a database is established, a user health state is calculated and determined. And then the terminal designs an APP to management the user health state.

Specifically, user's emotions and health states may be collected and analyzed by some sensors in a smart terminal to give out a determination, and then a terminal user would be reminded that health management has been performed. The application can achieve a better health management at a low cost on the basis of hardware and in combination with software.

In this embodiment, firstly, user health data is collected by various sensors, for example, judging the environment in which the human body is located and indirectly collecting emotional data or medical indicators; secondly, these data may be analyzed, compared and filtered to form a basic judgment, which is reflected on the APP of the terminal. For instance, an APP with health reminder is made to manage a user health state.

Specifically, some specific scene embodiments are listed as below.

Scene 1: scene A, at dusk, the user of the terminal holds this terminal which uses the method of present disclosure. The health management APP is started. Firstly, a light sensor may be started to collect that an illuminance is 100 Lux. Next, a camera sensor may be started to measure that the pupil size is 5 mm at that time. Assuming that a change coefficient of illuminance and pupil size Q= 0.002 mm/Lux, Y= (L-500)*Q = -0.8mm, A = X+Y=4.2mm. Assuming the standard pupil size B is 3.5 mm under the reference illuminance and in calmness, W = (A-B)/B= 20%. W is positive 20%, which represents that the corrected pupil size is enlarged by 20%. Then it can be judged that this terminal user is in exciting or pleasure, which is related to the user health state, is an event with high probability. Therefore, on the basis of the big database, a user health state can be managed in combination with the terminal APP. In such an embodiment, "A certain time at dusk in a certain place" and "User is mild excited or happy" will be recorded by a smart terminal and imported into the health management database, which can put forward some advices on health management. For instance, the terminal user may be reminded to go into this scene in the future at dusk to get a good mood again.

Scene 2: scene B, the sunshine is sufficient outdoor and the user holds the terminal which uses the method of the present disclosure. The health management APP is started. Firstly, a light sensor may be started to collect that illuminance is 900 Lux. Next, a camera sensor will be started to that pupil size is 2 mm at that time. Assuming that a change coefficient of illuminance and pupil size Q= 0.002 mm/Lux, then Y= (L-500)*Q = 0.8mm, A = X+Y=2.8mm. Assuming the standard pupil size B is 3.5 mm under the illuminance and in calmness, then W = (A-B)/B= -20%. Then W is -20%, meaning that the corrected pupil size is shrunk by 20%. Then it can be judged that this terminal user is depressed or tired, which is related to his/her health state, is an event with high probability. Therefore, on the basis of the big database, a user health state can be managed in combination with the terminal APP. In such an embodiment, "A certain time at daytime in a certain place" and "User is depressed or tired" will be recorded by a smart terminal and imported into the health management database, which can put forward some advices on health management. For instance, this terminal user would be reminded to not go into this scene in the future at daytime to avoid being in depressed and tired.

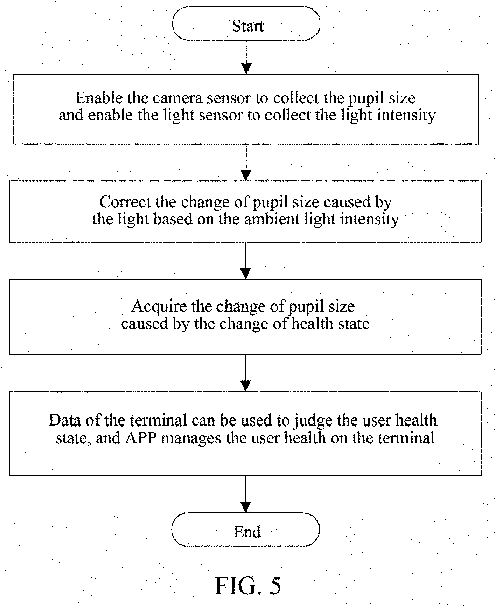

Fig.5 is a flow chart illustrating a method for monitoring user health according to a preferred embodiment of the present disclosure. As shown in Fig.5, a camera sensor is used as a sensor for collecting graphic images, in this embodiment, the graphic image is collected, the pupil size and its constant change are measured. A light sensor, used as a sensor for collecting ambient light, is used for capturing ambient light intensity (i.e., illuminance) in this embodiment. According to the ambient light intensity, the pupil may correct for changes in the size of the pupil caused by the ambient light. Specifically, if human eyes experience a greater illuminance, the pupil will have to shrink; while human eyes experience a less illuminance, the pupil will have to enlarge. The pupil diameter measured by a camera sensor is defined as X in millimeters; the pupil change caused by the illuminance is defined as Y in millimeters, and the corrected pupil size is defined as A in millimeters. Then, A = X + Y. The illuminance that can be felt by the human eyes is defined as L in lux, the reference illuminance is defined as 500 lux and the change coefficient of illuminance and pupil size is defined as Q in millimeters/lux. The coefficient Q comes from big data statistic, and represents by how many millimeters the pupil size may change when the illuminance changes by one 1 lux. Then, Y = (L-500)*Q. A = X + (L-500)*Q. A is a real pupil size reflecting a user health state. Supposing, the standard pupil size under the reference illuminance and in calmness is defined as B in millimeters, and the change rate of pupil size is defined as W, then W = (A-B) / B. According to the corrected change rate of pupil size and big data statistics, a database is established, a user health state can be calculated and determined. And then the terminal designs an APP to management the user health state.

Apparently, one skilled in the art should understand that each module or step of the disclosure described above may be implemented by using a general computing device which may be centralized on a single computing device or distributed over a network composed of computing devices. Optionally, they may be implemented by using program codes executable by the computing device, therefore, they can be stored in a storage device and executed by a computing device. In some cases, the steps shown or described can be performed in an order different from the order here, or they are made into individual integrated circuit modules, multiple of these modules or steps can be implemented as a single integrated circuit module. Thus, the present disclosure is not limited to any particular combination of hardware and software.

The description above is only for the preferred embodiments of the present disclosure and is not used for limiting the present disclosure. There can be various modifications and alterations of the present disclosure for the skilled in the art. Any modifications, equivalent substitutions and variations made with the essence and rule of the present disclosure would be included within the protection scope of the present disclosure.

### Industrial Applicability

Based on the technical scheme provided by embodiments of the present disclosure, the illuminance of the environment is acquired, where the user is currently located in the environment, the first pupil size is acquired when the user is located in the environment, the first pupil size is corrected on the basis of the illuminance to acquire a second pupil size; the second pupil size is compared with a reference pupil size to determine a change rate of a pupil size of the user; and the health state of the user is determined on the basis of the change rate of the pupil size. Thereby the problem that the terminal device may not monitor user health comprehensively can be solved and the effect of monitoring user's health comprehensively can be achieved.

## Claims

1. A method for monitoring user health, comprising:
Acquiring an illuminance of an environment, wherein the environment is an environment in which a user is currently located;
acquiring a first pupil size when the user is located in the environment;
correcting the first pupil size based on the illuminance to acquire a second pupil size;
comparing the second pupil size with a reference pupil size to determine a change rate of a pupil size of the user; and
determining a health state of the user based on the change rate of the pupil size.

2. The method according to claim 1, wherein correcting the first pupil size based on the illuminance to acquire a second pupil size comprises:
calculating a change size of pupil based on effects on the first pupil size under the illuminance;
calculating the second pupil size based on the change size of pupil and the first pupil size.

3. The method according to claim 2, wherein
calculating a change size of pupil based on effects on the first pupil size under the illuminance comprises: calculating the change size of pupil according to a formula *Y*=(*L*₁*-L*₂)**Q*, wherein *Y*=(*L*₁*-L*₂)**Q*, is the change size of pupil, *L*₁ is the illuminance, *L*₂ *is* a reference illuminance, *Q* is a change coefficient of pupil size;
calculating the second pupil size based on the change size of pupil and the first pupil size comprises: calculating the second pupil size according to a formula *A*=*X*+*Y,* wherein *A* is the second pupil size, *X* is the first pupil size.

4. The method according to claim 1, wherein comparing the second pupil size with a reference pupil size to determine a change rate of a pupil size of the user comprises:
calculating the change rate of the pupil size according to a formula *W*=(*A-B*)/*B*, wherein *W* is the change rate of the pupil size, *B* is the reference pupil size, *A* is the second pupil size.

5. The method according to claim 3, wherein determining a health state of the user based on the change rate of the pupil size comprises:
determining the health state of the user to be a first state when the change rate of the pupil size is greater than a preset threshold of change rate;
determining the health state of the user to be a second state when the change rate of the pupil size is less than the preset threshold of change rate.

6. The method according to claim 3, wherein determining a health state of the user based on the change rate of the pupil size further comprises:
determining the health state of the user to be a first state when the change rate of the pupil size is within a range of a first preset change rate;
determining the health state of the user to be a second state when the change rate of the pupil size is within a range of a second preset change rate;
determining the health state of the user to be a three state when the change rate of the pupil size is within a range of a three preset change rate.

7. The method according to claim 1, after determining a health state of the user based on the change rate of the pupil size, further comprising:
recording the health state of the user;
sending an alert message to a terminal held by the user, wherein the alert message comprises the health state of the user.

8. A device for monitoring user health, comprising:
a first acquisition module, configured to acquire an illuminance of an environment, wherein the environment is an environment in which a user is currently located;
a second acquisition module, configured to acquire a first pupil size when the user is located in the environment;
a correction module, configured to correct the first pupil size based on the illuminance to acquire a second pupil size;
a comparison module, configured to compare the second pupil size with a reference pupil size to determine a change rate of a pupil size of the user; and
a determination module, configured to determine a health state of the user based on the change rate of the pupil size.

9. The device according to claim 8, wherein the correction module comprises:
a first calculation unit, configured to calculate a change size of pupil based on effects on the first pupil size under the illuminance;
a second calculation unit, configured to calculate the second pupil size based on the change size of pupil and the first pupil size.

10. The device according to claim 9, wherein
the first calculation unit comprises: a first calculation subunit, configured to calculate the change size of pupil according to a formula *Y*=(*L*₁*-L*₂)**Q*, wherein *Y*=(*L*₁*-L*₂)**Q*, is the change size of pupil, *L*₁ is the illuminance, *L₂* is a reference illuminance, *Q* is a change coefficient of pupil size;
the second calculation unit comprises: a second calculation subunit, configured to calculate the second pupil size according to a formula *A*=*X*+*Y,* wherein *A* is the second pupil size, *X* is the first pupil size.

11. The device according to claim 8, wherein the comparison module comprises:
a third calculation unit, configured to calculate the change rate of the pupil size according to a formula *W*=(*A-B*)/*B,* wherein *W* is the change rate of the pupil size, *B* is the reference pupil size, *A* is the second pupil size.

12. The device according to claim 10, wherein the determination module comprises:
a first determination unit, configured to determine the health state of the user to be a first state when the change rate of the pupil size is greater than a preset threshold of change rate;
a second determination unit, configured to determine the health state of the user to be a second state when the change rate of the pupil size is less than the preset threshold of change rate.

13. The device according to claim 10, wherein the determination module further comprises:
a third determination unit, configured to determine the health state of the user to be a first state when the change rate of the pupil size is within a range of a first preset change rate;
a fourth determination unit, configured to determine the health state of the user to be a second state when the change rate of the pupil size is within a range of a second preset change rate;
a fifth determination unit, configured to determine the health state of the user to be a three state when the change rate of the pupil size is within a range of a three preset change rate.

14. The device according to claim 8, wherein the device further comprises:
a recording unit, configured to record the health state of the user;
a sending unit, configured to send an alert message to a terminal held by the user, wherein the alert message comprises the health state of the user.

15. A terminal for monitoring user health, comprising:
a first sensor, configured to acquire an illuminance of an environment, wherein the environment is an environment in which a user is currently located;
a second sensor, configured to acquire a first pupil size when the user is located in the environment;
a processor, connected with the first sensor and the second sensor, configured to correct the first pupil size based on the illuminance to acquire a second pupil size, compare the second pupil size with a reference pupil size to determine a change rate of a pupil size of the user and determine a health state of the user based on the change rate of the pupil size.
